# EUROPÄISCHE PATENTANMELDUNG

(11) **EP 2 263 526 A1**
(43) Veröffentlichungstag der Anmeldung: **22.12.2010**
(21) Anmeldenummer: 09008023.5
(22) Anmeldetag: 19.06.2009
(51) Int. Cl.: A61B 5/00, A61B 5/15

(54) **Stechsystem**

(71) Anmelder: Roche Diagnostics GmbH, 68305 Mannheim (DE); F. Hoffmann-La Roche AG, 4070 Basel (CH)
(72) Erfinder: Konya, Ahmet, 67165 Waldsee (DE)
(74) Vertreter: Mommer, Niels

(57) **Zusammenfassung**

Die Erfindung betrifft ein Stechsystem mit einem Trägerband (2), das Stechelemente (10) und zwischen den Stechelementen (10) angeordnete Testelemente (11) zur Untersuchung einer durch einen Stich gewonnenen Körperflüssigkeitsprobe trägt, einem Stechantrieb (9), um Stechelemente (10) in eine Stichbewegung zu versetzen, einen mit dem Stechantrieb (9) gekoppelten Trägerbandhalter (5), der einen Abschnitt des Trägerbandes (2) hält und sich bei einem Stich zusammen mit diesem Abschnitt (2a) und einem von dem Abschnitt (2a) getragenen Stechelement (10) in Stichrichtung bewegt, einer Aufwickelrolle (4), um das Trägerband (2) durch Aufwickeln in einer Bandtransportrichtung zu bewegen, einer Messeinrichtung (6) zur Messung einer Analytkonzentration einer von einem Testelement aufgenommenen Körperflüssigkeitsprobe. Erfindungsgemäß ist vorgesehen, dass ein Abschnitt (2b) des Trägerbandes (2) mit einem unbenutzten Testelement (11) an einem Trägerbandabschnitt (2a) vorbeigeführt ist, der im Trägerbandhalter (5) oder in Bandtransportrichtung hinter dem Trägerbandhalter (5) angeordnet ist, und zur Probenübergabe von einem Stechelement (10) auf ein Testelement (11) diese beiden Trägerbandabschnitte (2a, 2b) gegeneinander gedrückt werden. Die Erfindung betrifft ein ferner ein entsprechendes Verfahren zur Übergabe einer Körperflüssigkeitsprobe von einem Stechelement (10) auf ein Testelement (11).

## Beschreibung

Die Erfindung geht aus von einem Stechsystem mit den im Oberbegriff des Anspruchs 1 angegebenen Merkmalen.

Aus der WO 2008/083844 A1 ist ein derartiges Stechsystem bekannt, bei dem auf einem Trägerband zwischen Stechelementen jeweils ein Testelement angeordnet ist. Zum Erzeugen einer Stichwunde wird ein Trägerbandhalter zusammen mit einem ein Stechelement tragenden Trägerbandabschnitt in Stichrichtung bewegt. Zur Probenaufnahme wird das Trägerband weitertransportiert und anschließend der Trägerbandhalter erneut in Stichrichtung bewegt und so ein Testelement an eine zuvor mit einem Stechelement erzeugte Stichwunde herangeführt. Nachteilig hieran ist, dass für eine zuverlässige Probenaufnahme das Austreten einer relativ großen Probenmenge aus der Stichwunde erforderlich ist.

Aufgabe der Erfindung ist es, einen Weg aufzuzeigen, wie der Benutzerkomfort eines Stechsystems verbessert werden kann.

Diese Aufgabe wird durch ein Stechsystem mit den im Anspruch 1 angegebenen Merkmalen sowie durch ein Verfahren mit den im Anspruch 15 angegebenen Merkmalen gelöst. Vorteilhafte Weiterbildungen der Erfindung sind Gegenstand von Unteransprüchen.

Bei einem erfindungsgemäßen Stechsystem wird ein Abschnitt des Trägerbandes mit einem unbenutzten Testelement an einem im Trägerbandhalter oder in Bandtransportrichtung hinter dem Trägerbandhalter gehaltenen Trägerbandabschnitt vorbeigeführt. Zur Probenübergabe von einem Stechelement auf ein Testelement werden diese beiden Trägerbandabschnitte gegeneinander gedrückt.

Bei einem Einstich bleibt Körperflüssigkeit an einem Stechelement haften, die als Probe zur Bestimmung einer Analytkonzentration, beispielsweise der Glucosekonzentration, verwendet werden kann. Um die auf diese Weise gewonnene Körperflüssigkeitsmenge zu erhöhen, kann das Stechelement eine Probenaufnahmeeinrichtung, beispielsweise Vertiefungen, Durchbrüche oder einen Kapillarkanal in Form einer Rille oder eines Spalts, aufweisen.

Durch eine Probenübergabe von dem Stechelement zu einem Testelement kann vorteilhaft in kürzerer Zeit eine Analytkonzentration bestimmt werden, da mit einer einzigen Stichbewegung des Trägerbandhalters sowohl eine Stichwunde erzeugt als auch eine Körperflüssigkeitsprobe aufgenommen wird. Da bei einem erfindungsgemäßen Stechsystem kein selbstständiges Austreten von Körperflüssigkeit aus der Stichwunde erforderlich ist, kann zudem die Stichtiefe reduziert und damit eine schmerzärmere Probengewinnung realisiert werden.

Das eine Probe aufnehmende Testelement kann in Bandtransportrichtung vor oder hinter dem die Probe abgebenden Stechelement angeordnet sein. Bevorzugt ist das die Probe aufnehmende Testelement in Bandtransportrichtung aber hinter dem die Probe abgebenden Stechelement und damit hinter dem Trägerbandhalter angeordnet. Vorteilhaft kann auf diese Weise die Gefahr einer Verschmutzung des Trägerbandhalters durch Körperflüssigkeit reduziert werden.

Um einen Abschnitt des Trägerbandes mit einem unbenutzten Testelement an einem im Trägerbandhalter oder in Bandtransportrichtung hinter dem Trägerbandhalter gehaltenen Trägerbandabschnitt vorbeizuführen, kann eine Bandumlenkung, beispielsweise ein Stift oder eine Umlenkrolle, verwendet werden, die zwischen diesen beiden Trägerbandabschnitten angeordnet ist. Einer der beiden Trägerbandabschnitte führt dann in Bandtransportrichtung zu der Bandumlenkung hin der andere Trägerbandabschnitt von der Bandumlenkung weg. Die beiden Trägerbandabschnitte können auf diese Weise aneinander entlang, bevorzugt zueinander parallel, geführt werden.

Um die beiden vor der Aufwickelrolle angeordneten Trägerbandabschnitte gegeneinander zu drücken, können beide Trägerbandabschnitte aufeinander zu bewegt werden. Es genügt jedoch, einen der beiden Trägerbandabschnitt zu bewegen. Beispielsweise kann der das Stechelement tragende Trägerbandabschnitt zu dem das Testelement tragen Trägerbandabschnitt bewegt und gegen diesen gedrückt werden.

Vor einer Probenübergabe kann das Trägerband weitertransportiert werden, so dass sich das die Probe abgebende Stechelement bei der Probenübergabe nicht mehr in dem Trägerbandhalter befindet. Ein solcher Weitertransport ist aber nicht erforderlich. Bevorzugt erfolgt die Probenübergabe von einem in dem Trägerbandhalter angeordneten Stechelement auf ein Testelement, da auf diese Weise weniger Zeit zwischen einem Einstich und der Probenübergabe verstreicht, so dass mit einer Eintrocknung der Probe verbundene Probleme minimiert werden können.

Eine vorteilhafte Weiterbildung der Erfindung sieht vor, dass der Trägerbandhalter quer zur Stichrichtung beweglich ist. Durch eine entsprechende Bewegung kann zur Probenübergabe ein benutztes Stechelement gegen ein Testelement gedrückt werden, indem der Trägerbandhalter und damit der in ihm angeordnete Trägerbandabschnitt gegen den das unbenutzte Testfeld tragenden Trägerbandabschnitt gedrückt wird.

Die Stechelemente sind bei einem erfindungsgemäßen Stechsystem bevorzugt quer zur Längsrichtung orientiert, wie dies bei dem der WO 2008/083844 A1 bekannten System der Fall ist. Möglich ist es aber auch, die Stechelemente wie bei dem aus der US 2005/0245954 A1 bekannten System in Längsrichtung des Trägerbandes zu orientieren.

Ein erfindungsgemäßes Stechsystem wird bevorzugt von einem Stechgerät und einer in das Stechgerät einsetzbaren Bandkassette gebildet. Eine Bandkassette mit einer erfindungsgemäßen Bandführung ist Gegenstand des Anspruchs 14. Bevorzugt ist der Trägerbandhalter Teil der Bandkassette. In diesem Fall kann der Trägerbandhalter beim Einlegen der Bandkassette in ein Stechgerät an dessen Stechantrieb ankoppeln. Möglich ist es auch, dass der Trägerbandhalter Teil des Stechgeräts ist und beim Einlegen der Bandkassette in das Stechgerät einen Trägerbandabschnitt aufnimmt. Bevorzugt enthält die Bandkassette eine Vorratsrolle, auf der das unbenutzte Trägerband aufgewickelt ist. Das unbenutzte Trägerband kann in der Bandkassette aber auch zu einem Stapel gefaltet sein. Prinzipiell ist es aber auch möglich, ein erfindungsgemäßes Stechsystem als Wegwerfgerät zu realisieren, bei dem ein Austausch des Trägerbandes nicht vorgesehen ist und das bestimmungsgemäß entsorgt wird, sobald alle auf dem Trägerband angeordneten Stech- und Testelemente aufgebraucht sind.

Weitere Einzelheiten und Vorteile einer erfindungsgemäßen Stechsystems sowie eines erfindungsgemäßen Verfahrens zur Übergabe einer Körperflüssigkeitsprobe von einem auf einem Trägerband angeordneten Stechelement auf ein auf dem Trägerband angeordnetes Testelement werden an Ausführungsbeispielen unter Bezugnahme auf die beigefügten Zeichnungen erläutert. Gleiche und einander entsprechende Komponenten sind in den Zeichnungen mit übereinstimmenden Bezugszahlen bezeichnet. Es zeigen:
Figur 1 eine schematische Darstellung eines erfindungsgemäßen Stechsystems;
Figur 2 eine schematische Darstellung eines weiteren Ausführungsbeispiels; und
Figur 3 eine Seitenansicht zu Figur 2.

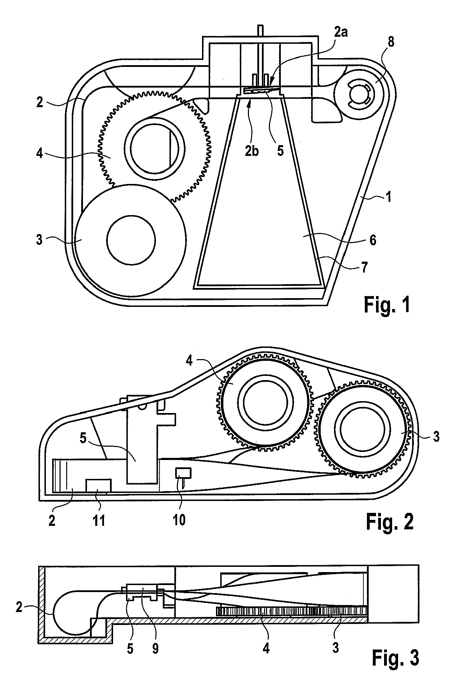

Figur 1 zeigt in einer schematischen Darstellung ein Ausführungsbeispiel eines Stechsystems bei geöffnetem Gerätegehäuse 1. In dem Gerätegehäuse 1 ist ein Trägerband 2 angeordnet, das Stechelemente und zwischen den Stechelementen angeordnete Testelemente zur Untersuchung einer durch einen Stich gewonnenen Körperflüssigkeitsprobe trägt. Das Trägerband 2 ist auf einer Vorratsrolle 3 aufgewickelt. Zum Gebrauch der Stechelemente und der Testelemente wird das Trägerband 2 schrittweise von der Vorratsrolle 3 abgewickelt und auf eine Aufwickelrolle 4 aufgewickelt, die auf diese Weise eine Bandtransporteinrichtung bildet.

Das Trägerband 1 wird von der Vorratsrolle 3 zu einem Trägerbandhalter 5 geführt, der mit einem in Figur 1 nicht dargestellten Stechantrieb gekoppelt ist und einen Abschnitt 2a des Trägerbandes 1 hält. Bei einem Stich bewegt sich der Trägerbandhalter 5 zusammen mit einem Stechelement, das der gehaltene Trägerbandabschnitt 2a trägt, in Stichrichtung. Bei dem dargestellten Ausführungsbeispiel sind die Stechelemente quer zur Längsrichtung des Trägerbandes orientiert, so dass die Stichrichtung senkrecht zur Zeichenebene der Figur 1 ist.

Die Analytkonzentration einer durch einen Stich gewonnen Körperflüssigkeitsprobe kann mit einer Messeinrichtung 6 gemessen werden. Die Messeinrichtung 6 wirkt dabei mit den Testelementen zusammen, die bevorzugt Nachweisreagenzien, beispielsweise für eine elektro-chemische oder photometrische Konzentrationsbestimmung enthalten. An sich kann die Messeinrichtung 6 aber auch für einen reagenzfreie Konzentrationsbestimmung, beispielsweise durch spektroskopische Analyse, vorgesehen sei. Bei dem dargestellten Ausführungsbeispiel ist die Messeinrichtung 6 für eine photometrische Konzentrationsbestimmung ausgebildet. Nachweisreagenzien der Testelemente bewirken hierfür eine konzentrationsabhängige Verfärbung, deren Intensität mit der Messeinrichtung 6 gemessen wird. Zur Minimierung von Streulicht hat die Messeinrichtung 6 ein Gehäuse 7, das von dem Gerätegehäuse 1 umgeben ist.

Bei einem Einstich in ein an eine Öffnung des Gerätegehäuses 1 angelegtes Körperteil eines Patienten, beispielsweise in einen Finger, bleibt Körperflüssigkeit an dem verwendeten Stechelement haften. Damit die so gewonnene Körperflüssigkeitsprobe untersucht werden kann, muss diese auf ein Testelement übertragen werden.

Bei dem dargestellten Ausführungsbeispiel werden zu diesem Zweck der Trägerbandabschnitt 2b mit einem unbenutzten Testelement und ein Trägerbandabschnitt 2a mit einem benutzen Stechelement gegeneinander gedrückt. Auf diese Weise wird ein Stechelement, an dem Körperflüssigkeit haftet, mit einem Testelement in Kontakt gebracht und so Körperflüssigkeit auf das Testelement übertragen.

Der Trägerbandabschnitt 2a mit dem die Probe abgegebenen Stechelement befindet sich bei dem gezeigten Ausführungsbeispiel in dem Trägerbandhalter 5. Der Trägerbandhalter 5 ist quer zur Stichrichtung und quer zur Längsrichtung des in ihm gehaltenen Trägerbandabschnitts 2a beweglich. Der Trägerbandhalter 5 kann deshalb zur Probenübergabe gegen einen an ihm vorbei geführten Trägerbandabschnitt 2b gedrückt werden. Bei dem dargestellten Ausführungsbeispiel wird der Trägerbandhalter 5 dabei auch gegen die Messeinrichtung 6, genauer gesagt gegen deren Gehäuse 7 gedrückt. Auf diese Weise kann ein Testfeld während einer Messung gegen die Messeinrichtung 6 gepresst werden, so dass definierte Bedingungen für eine präzise Messung vorliegen. Bevorzugt hat der Trägerbandhalter 5 auf einer Seite, die dem Trägerbandabschnitt 2b mit dem die Probe aufnehmenden Testfeld gegenüberliegt eine Aussparung. Vorteilhaft können so die beiden Trägerbandabschnitte 2a, 2b bei einer Probenübergabe flächig aneinander anliegen. Es genügt jedoch, wenn der Trägerbandhalter 5 das Trägerband 2 nur auf einem Teil seiner Breite hält und einen oberen Bereich mit den Spitzen der Stechelemente freilässt, so dass zur Probenübergabe die beiden Trägerbandabschnitte 2a, 2b mit ihrem oberen Bereich aneinander anliegen.

Prinzipiell kann das eine Probe aufnehmende Testelement in Bandtransportrichtung vor oder hinter dem Trägerbandhalter 5 angeordnet sein. Bei dem dargestellten Ausführungsbeispiel ist das eine Probe aufnehmende Testelement in Bandtransportrichtung hinter dem Trägerbandhalter 5 angeordnet. Vorteilhaft wird auf diese Weise die Gefahr einer Verschmutzung des Trägerbandhalters 5 durch Körperflüssigkeit vermieden.

Bei dem dargestellten Ausführungsbeispiel ist das Trägerband 2 von dem Trägerbandhalter 5 aus zu einer Umlenkrolle 8 und von dort an dem Trägerbandhalter 5 vorbei zu der Aufwickeleinrichtung 4 geführt. Auf diese Weise bildet das Trägerband 2 eine Schlaufe mit zwei aneinander entlang verlaufenden Trägerbandabschnitten, nämlich dem in dem Trägerbandhalter 5 gehaltenen Trägerbandabschnitt 2a und einem zwischen dem Trägerbandhalter 5 und der Messeinrichtung 6 verlaufenden Trägerbandabschnitt 2b. Zur Übergabe einer Probe von einem Stechelement auf ein Testfeld werden die beiden Trägerbandabschnitte 2a, 2b, die bevorzugt näherungsweise parallel verlaufen, zusammengedrückt, so dass das Stechelement das Testelement kontaktiert.

Die Länge der Bandschlaufe ist dabei passend zu dem Abstand von einem Stechelement zu dem ihm zugeordneten Testelement bemessen. Dabei ist es aber nicht erforderlich, dass die Länge der durch die Umlenkrolle 8 gebildeten Bandschlaufe dem Abstand zwischen einem Stechelement und dem benachbarten Testelement entspricht. Es ist nämlich auch möglich, dass eine Probenübergabe von einem Stechelement nicht zu einem benachbarten Testelement, sondern zu einem weiter entfernten angeordneten Stechelement, beispielsweise dem übernächsten oder überübernächsten Stechelement erfolgt.

Der Trägerbandhalter 5 kann im einfachsten Fall als ein Schlitz mit einer stets gleich bleibenden Breite ausgebildet sein. Bevorzugt weist der Trägerbandhalter 5 aber zwei gegeneinander bewegliche Teile auf, zwischen denen das Trägerband 2 hindurchgeführt ist. Auf diese Weise kann der Trägerbandhalter 5 das Trägerband 2 bei einem Stich zwischen den beiden gegeneinander beweglichen Teilen klemmend halten. Zum Bandtransport kann das Trägerband 2 freigegeben werden, also der Abstand zwischen den beiden gegeneinander beweglichen Teilen vergrößert werden, so dass mit geringer Bandreibung durch Aufwickeln der Aufwickelrolle 4 das nächste Stechelement in seine Gebrauchsposition in dem Trägerbandhalter 5 gebracht werden kann. Beispielsweise können die beiden gegeneinander beweglichen Teile des Trägerbandhalters 5 schwenkbar beweglich sein. Nach diesem Prinzip sind auch die zusammen wirkenden Schenkel einer Zange beweglich, um etwas klemmend zu halten.

Bevorzugt erfolgt zwischen einem Stich und einer Probenübergabe auf ein Testelement kein Bandtransport, d. h. die Aufwickelrolle 4 wird zwischen einem Stich und einer Probenübergabe nicht bewegt. Die Gebrauchsposition eines Stechelements in dem Trägerbandhalter 5, die es bei einem Stich inne hat, wird demnach bevorzugt auch zur Probenübergabe genutzt.

Die Bewegung des Trägerbandhalters 5 zum Zusammenpressen der beiden Trägerbandabschnitte 2a, 2b bei einer Probenübergabe wird bevorzugt von dem Stechantrieb im Anschluss an eine Stichbewegung selbsttätig bewirkt. Beispielsweise kann der Stechantrieb hierfür eine geeignete Kulissensteuerung aufweisen. Es ist nämlich vorteilhaft, wenn eine Probe sobald wie möglich von einem Stechelement an ein Testelement übergeben wird. Insbesondere bei trockener Umgebungsluft besteht nämlich die Gefahr, dass Körperflüssigkeit verdunstet und eine Probe am Stechelement eintrocknet, so dass eine Probenübergabe nur in einem kurzen Zeitfenster möglich ist.

Die Figuren 2 und 3 zeigen ein weiteres Ausführungsbeispiels bei geöffnetem Gerätegehäuse 1 in einer schematischen Draufsicht und eine dazugehörenden Seitenansicht, in welcher auch der mit dem Trägerbandhalter 5 gekoppelte Stechantrieb 9 dargestellt ist. Der Unterschied zu dem vorstehend beschriebenen Ausführungsbeispiel besteht dabei im Wesentlichen darin, dass das Trägerband 2 auf seinem Weg von der Vorratsrolle 3 zu dem Trägerbandhalter 5 eine Vierteldrehung durchführt. Die geometrische Drehachse der Vorratsrolle 3 ist deshalb nicht wie bei dem vorstehend beschriebenen Ausführungsbeispiel parallel zur Stichrichtung, sondern quer dazu orientiert.

Zwischen der Messeinrichtung 6 und der Aufwickelrolle 4 ist das Trägerband 2 um eine weitere Vierteldrehung verdrillt. Die geometrische Drehachse der Aufwickelrolle 4 ist deshalb parallel zur geometrischen Drehachse der Vorratsrolle 3 orientiert. Die zweite Vierteldrehung des Trägerbandes 2 kann die vorhergehende Vierteldrehung rückgängig machen oder sich dazu addieren, so dass das Trägerband 2 insgesamt um eine halbe Drehung verdrillt ist.

In Figur 2 sind die in Figur 1 nicht dargestellten Stechelemente 10 und Testelemente 11 auf dem Trägerband 2 zu erkennen. Die Stechelemente 10 können aus Metall, insbesondre Stahl, hergestellt werde, wobei jedoch auch andere Materialien wie Kunststoff oder Keramik verwendet werden können. Die Stechelemente 10 haben eine Probenaufnahmeeinrichtung, die beispielsweise als ein Kapillarkanal oder Vertiefungen bzw. Durchbrüche, in denen Körperflüssigkeit bei einem Stich haften bleibt, ausgebildet sein kann. Ein Kapillarkanal ist ein Kanal, in dem Körperflüssigkeit bei einem Stich durch Kapillarkräfte gehalten wird. Ein solcher Kanal kann als eine Rinne ausgebildet sein oder ein Schlitz sein, der zur Ober- und Unterseite des Stechelements 10 hin offen ist.

Die Testelemente 11 können als Testfelder auf dem Trägerband 2 vorgesehen sein. Die Testelemente 11 haben eine saugfähige Oberfläche, um eine Probenaufnahme von einem Stechelement 10 zu erleichtern. Die saugfähige Oberfläche kann beispielsweise durch Auftragen einer Paste oder durch ein Vlies gebildet werden und Nachweisreagenzien enthalten. Für eine photometrische Konzentrationsbestimmung kann es vorteilhaft sein, ein transparentes Trägerband 2 zu verwenden, so dass Messlicht durch das Trägerband 2 hindurch gelangen kann. Möglich ist es auch, Testelemente auf passende Fenster des Trägerbandes 2 aufzukleben.

### Bezugszahlen

- 1: Gerätegehäuse
- 2: Trägerband
- 2a: Trägerbandabschnitt
- 2b: Trägerbandabschnitt
- 3: Vorratsrolle
- 4: Aufwickelrolle
- 5: Trägerbandhalter
- 6: Messeinrichtung
- 7: Gehäuse
- 8: Umlenkrolle
- 9: Stechantrieb
- 10: Stechelement
- 11: Testelement

## Patentansprüche

1. Stechsystem mit
einem Trägerband (2), das Stechelemente (10) und zwischen den Stechelementen (10) angeordnete Testelemente (11) zur Untersuchung einer durch einen Stich gewonnenen Körperflüssigkeitsprobe trägt,
einem Stechantrieb (9), um Stechelemente (10) in eine Stichbewegung zu versetzen,
einen mit dem Stechantrieb (9) gekoppelten Trägerbandhalter (5), der einen Abschnitt des Trägerbandes (2) hält und sich bei einem Stich zusammen mit diesem Abschnitt (2a) und einem von dem Abschnitt (2a) getragenen Stechelement (10) in Stichrichtung bewegt,
einer Aufwickelrolle (4), um das Trägerband (2) durch Aufwickeln in einer Bandtransportrichtung zu bewegen,
einer Messeinrichtung (6) zur Messung einer Analytkonzentration einer von einem Testelement aufgenommenen Körperflüssigkeitsprobe,
**dadurch gekennzeichnet, dass**
ein Abschnitt (2b) des Trägerbandes (2) mit einem unbenutzten Testelement (11) an einem Trägerbandabschnitt (2a) vorbeigeführt ist, der im Trägerbandhalter (5) oder in Bandtransportrichtung hinter dem Trägerbandhalter (5) angeordnet ist, und zur Probenübergabe von einem Stechelement (10) auf ein Testelement (11) diese beiden Trägerbandabschnitte (2a, 2b) gegeneinander gedrückt werden.

2. Stechsystem nach Anspruch 1, **dadurch gekennzeichnet, dass** die beiden Trägerbandabschnitte (2a, 2b) während einer Messung gegeneinander gedrückt werden.

3. Stechsystem nach einem der vorstehenden Ansprüche, **dadurch gekennzeichnet, dass** das Trägerband (2) zwischen den beiden Trägerbandabschnitten(2a, 2b), die zur Probenübergabe gegeneinander gedrückt werden, eine Schlaufe bildet.

4. Stechsystem nach einem der vorstehenden Ansprüche, **dadurch gekennzeichnet, dass** das Trägerband (2) zwischen den beiden Trägerbandabschnitten (2a, 2b), die zur Probenübergabe gegeneinander gedrückt werden, um eine Umlenkrolle (8) geführt ist.

5. Stechsystem nach einem der vorstehenden Ansprüche, **dadurch gekennzeichnet, dass** die Probenübergabe von einem Stechelement (10) zu einem Testelement (11) erfolgt, das in Bandtransportrichtung vor dem Stechelement (10) auf dem Trägerband (2) angeordnet ist.

6. Stechsystem nach einem der vorstehenden Ansprüche, **dadurch gekennzeichnet, dass** zur Probenübergabe ein das Stechelement (10) tragender Trägerbandabschnitt (2a) zu einem ein Testelement (11) tragenden Bandabschnitt (2b) hin bewegt wird.

7. Stechsystem nach einem der vorstehenden Ansprüche, **dadurch gekennzeichnet, dass** die Probenübergabe von einem in dem Trägerbandhalter (5) angeordneten Stechelement (10) auf ein Testelement (11) erfolgt.

8. Stechsystem nach einem der vorstehenden Ansprüche, **dadurch gekennzeichnet, dass** der Trägerbandhalter (5) quer zur Stichrichtung beweglich ist, um zur Probenübergabe ein benutztes Stechelement (10) gegen ein Testelement (11) zu drücken.

9. Stechsystem nach einem der vorstehenden Ansprüche, **dadurch gekennzeichnet, dass** die Stechelemente (10) quer zur Längsrichtung des Trägerbands (2) orientiert sind.

10. Stechsystem nach einem der vorstehenden Ansprüche, **dadurch gekennzeichnet, dass** der Trägerbandhalter (5) zwei gegeneinander bewegliche Teile aufweist, zwischen denen das Trägerband (2) hindurchgeführt ist.

11. Stechsystem nach Anspruch 10, **dadurch gekennzeichnet, dass** der Trägerbandhalter (5) das Trägerband (2) bei einem Stich zwischen den beiden gegeneinander beweglichen Teilen klemmt.

12. Stechsystem nach einem der vorstehenden Ansprüche, **dadurch gekennzeichnet, dass** das Trägerband (2) bei einer Messung gegen die Messeinrichtung (6) gedrückt wird.

13. Stechsystem nach Anspruch 12, **dadurch gekennzeichnet, dass** das Trägerband (2) von dem Trägerbandhalter (5) gegen das Gehäuse (7) der Messeinrichtung gedrückt wird.

14. Bandkassette für ein Stechsystem nach einem der vorstehenden Ansprüche, die ein Trägerband (2), das Stechelemente (10) und zwischen den Stechelementen (10) angeordnete Testelemente (11) zur Untersuchung einer durch einen Stich gewonnenen Körperflüssigkeitsprobe trägt, und eine Aufwickelrolle (4) zum Aufwickeln des Trägerbandes (2) enthält, **dadurch gekennzeichnet, dass** zwei Trägerbandabschnitte (2a, 2b) aneinander vorbeigeführt sind, so dass zur Übergabe einer Probe von einem Stechelement (10) auf ein Testelement (11) diese beiden Trägerbandabschnitte (2a, 2b) gegeneinander gedrückt werden können.

15. Verfahren zur Übergabe einer Körperflüssigkeitsprobe von einem auf einem Trägerband (2) angeordneten Stechelement (10) auf ein auf dem Trägerband (2) angeordnetes Testelement (11), **dadurch gekennzeichnet, dass** das Trägerband (2) um eine Bandumlenkung (8) geführt ist, die zwischen einem das Stechelement (10) tragenden Trägerbandabschnitt (2a) und einem das Testelement (11) tragenden Trägerbandabschnitt (2b) angeordnet ist, und die beiden Trägerbandabschnitt (2a, 2b) zur Probenübergabe zusammengedrückt werden, so dass das Stechelement (10) das Testelement (11) kontaktiert.
